# EUROPÄISCHE PATENTANMELDUNG

(11) **EP 3 141 271 A1**
(43) Veröffentlichungstag der Anmeldung: **15.03.2017**
(21) Anmeldenummer: 15184862.9
(22) Anmeldetag: 11.09.2015
(51) Int. Cl.: A61M 1/12, A61M 1/10

(54) **BLUTPUMPE, VORZUGSWEISE ZUR UNTERSTÜTZUNG EINES HERZENS**

(71) Anmelder: Berlin Heart GmbH, 12247 Berlin (DE)
(72) Erfinder: NÜSSER, Peter, 14532 Kleinmachnow (DE); CHOUB, Leonid, 10585 Berlin (DE); GRAICHEN, Kurt, 13189 Berlin (DE); MÜLLER, Jörg, 10439 Berlin (DE)
(74) Vertreter: Pfenning, Meinig & Partner mbB

(57) **Zusammenfassung**

Gegenstand der vorliegenden Anmeldung ist eine Blutpumpe, vorzugsweise zur Unterstützung eines Herzens, wobei die Blutpumpe ein Gehäuse mit einem distalen Einlass, einem proximalen Auslass sowie einem zwischen dem Einlass und dem Auslass angeordneten Dorn umfasst. Koaxial auf dem Dorn ist ein Rotor mit Beschaufelung drehend angeordnet. Der Rotor ist auf dem Dorn in einer axialen Richtung magnetisch gelagert. In einer Ausführungsform befindet sich der Auslass proximal eines proximalen Endes der Beschaufelung des Rotors.

## Beschreibung

Gegenstand der vorliegenden Anmeldung ist eine Blutpumpe gemäß dem Oberbegriff des Anspruchs 1.

Blutpumpen, beispielsweise zur Unterstützung eines Herzens, werden beispielsweise bei Patienten mit Herz- oder Gefäßschwächen eingesetzt. Die in dieser Anmeldung beschriebenen Blutpumpen können beispielsweise als Ventricular Assist Devices (VADs) zur Unterstützung des linken Ventrikels, des rechten Ventrikels oder in einem System mit zwei Pumpen für beide Ventrikel eingesetzt werden.

Beispielsweise beschreibt die US 2014/0322020 ein VAD mit einer Radialpumpe. Radialpumpen sind dadurch gekennzeichnet, dass sie einen Rotor mit einer Beschaufelung aufweisen, wobei sich die Beschaufelung von einem Pumpeneinlass zu einem Pumpenauslass hin radial erstreckt. Aufgrund der Form des Rotors und der darauf angeordneten Beschaufelung wird das durch den Rotor transportierte Blut bewegt, wobei der Fördervorgang und die damit verbundene Blutschädigung von der Form des Rotors und der darauf angeordneten Beschaufelung abhängen.

Eine weitere Blutpumpe ist in der US 2006/0024182 gezeigt. Auch hier handelt es sich um eine Radialpumpe, bei welcher ein Motor, der proximal des Rotors liegt, den Rotor mit einem Drehmoment beaufschlagt und so in Drehung versetzt. Nachteil einer derartigen Konstruktion ist es, dass der Motor eine Vielzahl von rotierenden Komponenten neben dem Rotor aufweist.

Aufgabe des Gegenstandes der vorliegenden Anmeldung ist es, eine Blutpumpe zur Verfügung zu stellen, welche einfacher aufgebaut ist und mit welcher Blut bei geringer Blutschädigung gefördert werden kann.

Erfindungsgemäß wird die Aufgabe durch eine Blutpumpe beispielsweise gemäß Anspruch 1 gelöst.

Die Blutpumpe umfasst dabei ein Gehäuse mit einem stromaufwärts gelegenen (im Folgenden auch: distalen) Einlass sowie einem stromabwärts gelegenen (im Folgenden auch: proximalen) Auslass und einem zwischen dem Einlass und dem Auslass angeordneten Dorn. Der Dorn erstreckt sich dabei vom stromabwärts gelegenen Ende der Pumpe stromaufwärts im Inneren des Gehäuses zwischen dem stromaufwärts gelegenen Einlass und dem stromabwärts gelegenen Auslass. Koaxial ist auf dem Dorn ein Rotor mit einer Achse und einer Beschaufelung drehend angeordnet, wobei der Rotor auf dem Dorn in einer axialen Richtung magnetisch gelagert ist. Bei den nachfolgend verwendeten Begriffen distal und proximal wird keine Einschränkung hinsichtlich der Einbaulage der Pumpe gemacht, sondern lediglich eine Aussage zu der Anordnung einzelner Komponenten zueinander gemacht.

Der Auslass ist dabei stromabwärts eines stromabwärts gelegenen Endes der Beschaufelung angeordnet, bzw. der Rotor ist derart im Gehäuse angeordnet, dass eine Beschaufelung stromaufwärts des stromabwärts gelegenen Auslasses endet.

Eine Blutpumpe mit solchen Merkmalen ist also derart beschaffen, dass zunächst der Rotor das Blut zwischen dem Einlass und proximalen Auslass mit einer axialen Komponente fördert, wobei die Beschaufelung proximal des Auslasses endet. Anschließend wird das Blut durch das nachströmende geförderte Blut in Richtung des Auslasses und durch den Auslass der Pumpe in Richtung eines (Blut-)Gefäßes gepumpt.

Dadurch, dass der Rotor auf dem Dorn in der axialen Richtung magnetisch gelagert ist, kann je nach Einsatzgebiet der Pumpe ein aktives Lager oder ein passives Lager axial ausgebildet werden. Bei einem axialen, passiven magnetischen Lager werden Permanentmagnete sowohl im Rotor als auch im Pumpengehäuse bzw. im Dorn angeordnet und derart zueinander ausgerichtet, dass eine Bewegung des Rotors in Richtung des distalen Pulpeneinlasses aufgrund steigender Magnetkräfte bei einer Bewegung des Rotors in distaler Richtung verhindert wird. Beispielsweise können die Permanentmagnete eine Magnetisierung parallel zur Achse des Dornes aufweisen.

Bei der Verwendung eines aktiven axialen magnetischen Lagers interagieren Permanentmagnete des Rotors mit einer Steuerspule. Ein Stromfluss durch die Steuerspule verändert das magnetische Feld und kann die Permanentmagnete des Rotors anziehen oder abstoßen. Die Position des Rotors wird beispielsweise mit Hilfe einer Sensorspule ermittelt und ein etwaiger Sollabstand bzw. eine etwaige Sollposition des Rotors (in Abhängigkeit vom Arbeitspunkt der Pumpe) durch Beaufschlagung der Steuerspule mit einem entsprechenden Strom hergestellt. Magnetische Lager haben sich als besonders wartungsarm herausgestellt.

Der Dorn kann eine Funktion der radialen Lagerung des Rotors übernehmen. So verhindert der Dorn beispielsweise, dass in radialer Richtung beliebige Positionen durch den Rotor innerhalb des Gehäuses eingenommen werden können. Zudem kann der Dorn in einigen Ausführungsbeispielen Komponenten eines Stators zum Antrieb des Rotors enthalten. Zusätzlich oder alternativ können im Dorn Permanentmagnete, beispielsweise in Form von Magnetringen, angeordnet sein, welche einen Teil der magnetischen, axialen Lagerung bilden. In anderen Ausführungsbeispielen können beispielsweise Elemente des Stators, welche den Rotor in Drehung versetzen, auch innerhalb des Gehäuses angeordnet werden, so dass diese Stator-Komponenten den Rotor radial umgeben. Hinsichtlich des Dorns, des Einlasses und des Auslasses wird beispielsweise auf die Druckschrift WO 2012/150045 verwiesen, welche eine Herzpumpe mit einem Dorn zeigt, auf welchem ein Rotor angeordnet ist. Hierbei ist jedoch zu beachten, dass in jener Druckschrift die Beschaufelung nicht distal des Auslasses zu enden scheint.

Weitere Ausführungsbeispiele werden nachfolgend und in den Figuren beschrieben.

In einer Ausführungsform ist der Auslass vollständig proximal eines proximalen Endes der Beschaufelung angeordnet. Dies ist so zu verstehen, dass zwischen dem proximalen Ende der Beschaufelung des Rotors und dem distalen Beginn des Auslasses ein von null verschiedener, positiver Abstand besteht. Ausgehend von den Pumpenparametern, der Drehzahl, Rotorgröße, Beschaufelungsgröße und Ähnlichem, kann der Abstand so bestimmt werden, dass die Blutschädigung minimiert wird. In einigen Ausführungsbeispielen ist der Abstand zwischen dem Auslass und der Beschaufelung größer als 0,05 mm, vorzugsweise größer als 0,1 mm, 0,5 mm, 1 mm bzw. 3 mm, und kleiner als 10 mm, vorzugsweise kleiner als 5 mm. Bezüglich der Bestimmung des Abstandes zwischen dem proximalen Ende der Beschaufelung und dem proximalen Auslass kann der Abstand in einigen Ausführungsbeispielen zwischen dem distalsten Punkt des proximalen Auslasses, projiziert auf die Achse des Dorns bzw. Rotors, und dem proximalen Ende der Beschaufelung, projiziert auf die Achse des Dorns bzw. Rotors, gemessen werden. In anderen Ausführungsbeispielen kann der Abstand zwischen einem distalsten Punkt einer Spiralkammer und dem proximalen Ende der Beschaufelung, jeweils projiziert auf die Achse des Dorns bzw. Rotors, gemessen werden. Unter einer Spiralkammer bzw. Tangentialkammer kann hierbei ein Bereich des Gehäuses verstanden werden, welcher sich an einen beispielsweise zylinderförmigen Grundkörper anschließt und welcher derart geformt ist (beispielsweise als Schneckenform), dass dem Blut aufgrund der Gehäusegeometrie eine Tangentialkomponente der Strömungsgeschwindigkeit aufgezwungen wird. Dabei kann der Übergang vom Grundkörper zur Volute stetig oder sprunghaft verlaufen, in dem Sinne, dass ein Durchmesser des Grundkörpers sich stetig oder sprunghaft zu einem größeren Durchmesser der Spiralkammer aufweitet.

In einer besonders bevorzugten Variante ist der Auslass gegenüber der Achse des Rotors um einen Winkel von mehr als 45°, vorzugsweise zwischen 80° und 100°, geneigt. Insbesondere kann der Auslass ein Spiralgehäuse (oder auch: Volute) umfassen, dessen Auslassrichtung im Wesentlichen um 90° von der Achse des Rotors abweicht. Der Auslass kann dabei beispielsweise als ein Spiralgehäuse ausgebildet sein, wie dies beispielsweise in der WO 2012/150045 dargestellt ist.

In einer weiteren Ausführungsform umfasst der Rotor eine Hülse, insbesondere eine Zylinderhülse, wobei die Beschaufelung entlang eines äußeren Umfangs der Hülse angeordnet ist. Die Zylinderhülsenform des Rotors hat die Vorteile, dass ebenfalls ein zylinderförmiger Dorn verwendet werden kann. Sowohl die Zylinderhülse als auch ein zylinderförmiger Dorn haben den Vorteil, dass diese Geometrie vergleichsweise einfach herzustellen ist und somit die Pumpen kostengünstig herstellbar sind. Zudem hat sich gezeigt, dass die Auslegung der Lagerung des Motors durch die Zylinderhülse vereinfacht wird. Eine Zylinderhülse bietet auch die Möglichkeit, einen Hohlraum zwischen einer Zylinderinnenwand und einer Zylinderaußenwand zu bilden, in welchem beispielsweise Permanentmagnete für ein axiales Magnetlager angeordnet werden können oder sind.

In einer weiteren Ausführungsform befindet sich zwischen der Hülse und dem Dorn ein hydrodynamisches Lager. Dabei ist der Spalt zwischen der Oberfläche des Dorns und einem inneren Umfang des Rotors bzw. der Hülse bzw. der Zylinderhülse derart bemessen und ausgestaltet, dass den Spalt durchströmendes Blut den Rotor radial stabilisiert und somit ein radiales Lager bildet. Dabei kann das radiale Lager beispielsweise zwischen zwei im Wesentlichen parallelen Oberflächen des Rotors bzw. des Dorns ausgebildet sein, deren Abstand derart gewählt ist, dass das hindurchströmende Blut eine Gleitfläche bildet. In anderen Varianten kann entweder die Oberfläche des Dorns und/oder die Innenfläche des Rotors mit Kanälen oder ähnlichen Strukturen beaufschlagt sein, um das hydrodynamische Lager auszubilden.

Hinsichtlich der hydrodynamischen Lagerung wird beispielsweise auf die WO 2012/150045 verwiesen.

In einer weiteren Ausführungsform ist die Beschaufelung als eine Wendel ausgebildet, Unter einer Wendel wird hierbei eine helixartige Struktur verstanden, welche beispielsweise auf einem Zylindermantel abgewickelt ist. Dabei kann der Rotor lediglich eine Wendel oder mehrere Wendeln umfassen. In zahlreichen Ausführungsbeispielen besitzt die Wendel dabei eine im Querschnitt betrachtete Breite, welche deutlich kleiner ist als der Abstand zweier Wendeln auf der Rotoroberfläche. Ein Beispiel einer Beschaufelung ist beispielsweise die archimedische Schraube. Dies bedeutet, dass die Breite der Flächen, durch welche das Blut gefördert wird, größer ist als die Breite der Wendel. Die Flüssigkeit wird quasi in Richtung des Auslasses "geschraubt". Hinsichtlich der Wendel wird ebenfalls auf die WO 2012/150045 verwiesen,

In einer weiteren Ausführungsform ist der Rotor derart ausgebildet, dass ein proximales Ende des Rotors proximal des proximalen Endes der Beschaufelung des Rotors liegt. In anderen Worten erstreckt sich der Rotor in proximaler Richtung weiter als die Beschaufelung. Es existiert also ein proximaler Abschnitt des Rotors, welcher nicht mit einer Beschaufelung versehen ist, Ein derartiger Rotor ermöglicht beispielsweise eine sehr einfache Ausgestaltung des Dorns. Der Dorn muss hierbei keinerlei Strukturierungen aufweisen.

In weiteren Ausführungsformen umfasst der Dorn einen ersten distalen Abschnitt zur Aufnahme des Rotors und einen zweiten, proximal des ersten Abschnitts gelegenen Abschnitt, wobei zwischen dem ersten und zweiten Abschnitt eine Schulter einen Anschlag für ein proximales Ende des Rotors bildet. In dieser Ausführungsform kann der Rotor bis zu seinem proximalen Ende hin mit einer Beschaufelung versehen sein. Aufgrund der Schulter, welche distal des proximalen Auslasses liegt, endet auch die Beschaufelung des Rotors distal des proximalen Auslasses. Durch die Schulter wird zum einen ein mechanischer Anschlag des Rotors ausgebildet, und ferner kann im Betrieb der Pumpe zwischen der Schulter und dem Rotor ein hydrodynamischer Druck aufgebaut werden, welcher ein Anschlagen des Rotors an der Schulter verhindert.

In einer weiteren Ausführungsform besitzt der Dorn eine im Wesentlichen ringförmige Ausnehmung, in welcher der Rotor drehbar gelagert ist. Dies hat beispielsweise den Vorteil, dass der Rotor mechanisch in axialer Richtung nicht ausbrechen kann. Nachteilig ist jedoch der erschwerte Zusammenbau der Blutpumpe. In einigen Varianten des Vorhandenseins einer ringförmigen Ausnehmung ist die Beschaufelung des Rotors radial außerhalb der Ausnehmung angeordnet.

Der Gegenstand der Anmeldung soll anhand der in den Figuren beschriebenen Elemente näher beschrieben werden. Es zeigen
- Fig. 1: eine Darstellung einer Verwendung einer Blutpumpe als VAD;
- Fig. 2: eine schematische Darstellung einer Blutpumpe im Längsschnitt;
- Fig. 3: eine schematische Darstellung der Blutpumpe der Fig. 2 im Querschnitt;
- Fig. 4 und Fig. 5: Ausführungsformen einer Blutpumpe gemäß der Anmeldung mit zylinderförmigem Dorn;
- Fig. 6: einen Längsschnitt einer Ausführungsform einer Blutpumpe mit einer ringförmigen Ausnehmung;
- Fig. 7 und Fig. 8: Ausführungsformen einer Blutpumpe mit einer an dem Dorn angeordneten Schulter.

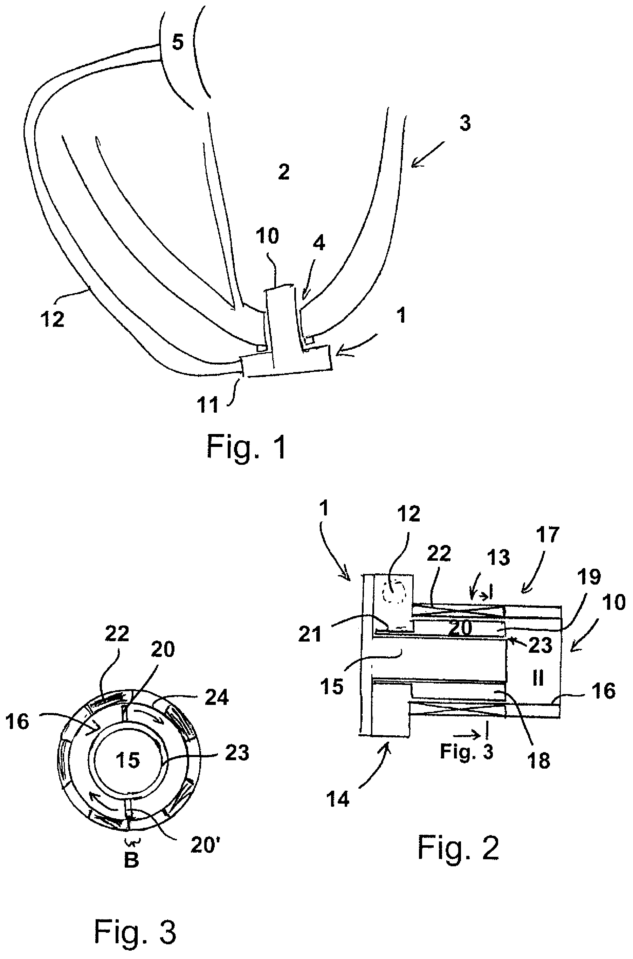

Fig. 1 zeigt eine Blutpumpe 1, welche in einem linken Ventrikel 2 eines Herzens 3 angeordnet ist. Dabei ragt ein Einlass 10 durch ein Loch im Apex 4 des Herzens, so dass Blut durch den Einlass 10 der Blutpumpe 1 zugeführt wird und durch den proximalen Auslass 11 über einen Graft oder eine Kanüle 12 einem weiteren Gefäß, wie beispielsweise einer Aorta 5, zugeführt werden kann. Obgleich die Blutpumpe 1 im vorliegenden Ausführungsbeispiel im linken Ventrikel 2 angeordnet ist und somit ein LVAD (left VAD) bildet, kann sie in anderen Ausführungsformen auch als RVAD (right VAD) oder in Kombination mit einer weiteren Blutpumpe als BiVAD (biventricular assist device) konzipiert werden.

Die Blutpumpe 1 umfasst weitere Elemente, welche hier zur Vereinfachung nicht dargestellt sind. So umfasst die Blutpumpe beispielsweise eine Kontrolleinheit, welche im Pumpengehäuse oder außerhalb des menschlichen oder tierischen Körpers angeordnet sein kann. Zudem ist die Blutpumpe mit einem Energieversorgungssystem verbunden, so dass die Pumpe mit ausreichender Energie zum Betrieb versehen wird. Weiterhin kann die Blutpumpe eine "Drive-Line" umfassen, über welche Steuersignale von der Pumpe zur extrakorporalen Kontrolleinheit oder die Stromversorgung von der extrakorporalen Versorgungseinheit zur Blutpumpe 1 geführt werden können.

Obgleich in der vorliegenden Anmeldung nicht näher ausgeführt, kann die Blutpumpe 1 in ihrem Gehäuse Elektronik umfassen. Hiermit können beispielsweise Sensordaten, welche als analoge Signale vorliegen, digitalisiert und so der Kontrolleinheit zugeführt werden.

In der Fig. 2 ist die exemplarische Blutpumpe 1 der Fig. 1 in einem Längsschnitt dargestellt. Wie bereits anhand der Fig. 1 beschrieben, umfasst die Blutpumpe 1 einen distalen Einlass 10 und einen proximalen Auslass 11. Zwischen dem Einlass 10 und dem Auslass 11 erstreckt sich ein Hohlraum 12, welcher sich zwischen einem distalen zylindrischen Abschnitt 13 und einem Spiralauslass 14 im Bereich des Auslasses 11 erstreckt. In den Spiralauslass 14 und den distalen Abschnitt 13 ragt ein Dorn 15 vom proximalen Ende zum distalen Ende hin. Der Dorn ist dabei derart beschaffen, dass zwischen einer Innenwand 16 des distalen Abschnitts des Gehäuses 17 und dem Dorn 15 ein ausreichend großer Spalt verbleibt, um einen Rotor 18 auf den Dorn 15 aufstecken zu können. Der Rotor 18 umfasst einen distalen Abschnitt 19 mit einer Beschaufelung 20. Weiterhin ist ein proximaler Abschnitt 21 dargestellt, welcher sich an den distalen Abschnitt 19 anschließt. Der proximale Abschnitt 21, welcher in die Spiralkammer 14 ragt, weist dabei keine Beschaufelung auf, so dass die Beschaufelung 20 distal des proximalen Auslasses 11 und distal der Spiralkammer 14 endet. Im vorliegenden Ausführungsbeispiel ist ein Stator zum Versetzen des Rotors in eine Drehbewegung innerhalb eines Hohlraums 12 angeordnet. Der Stator 22 umläuft den Rotor 18 und ist in einem Spalt oder Hohlraum des Gehäuses angeordnet.

Zwischen dem Rotor 18 und dem Dorn 15 befindet sich ein radialer Spalt 23, welcher derart beschaffen ist, dass sich in diesem Spalt ein Blutfilm ausbildet, sobald der Rotor in Bewegung versetzt wird, und der Rotor 18 auf diesem Blutfilm gelagert wird. Auf diese Weise bildet der radiale Spalt 23 ein hydrodynamisches Lager. Dabei ist die Blutflussrichtung innerhalb des Spalts 23 in einigen Ausführungsbeispielen der Förderrichtung des Blutes durch die Pumpe gegenläufig.

Zwischen einem radial äußeren Durchmesser des Rotors 18, insbesondere einem radial äußeren Durchmesser der Beschaufelung 20, und der Innenwand 16 des Gehäuses 17 befindet sich ein Spalt, welcher in einigen Ausführungsbeispielen kein hydrodynamisches Lager bildet.

Der Dorn 15 ragt in den distalen Abschnitt 13 der Blutpumpe 1. An seinem distalen Ende ist der Dorn 15 im vorliegenden Beispiel flach ausgeführt. In anderen Ausführungsbeispielen kann der Dorn an seinem distalen Ende gerundet oder sich aufweitend ausgebildet sein. Dies kann den Strömungswiderstand des Blutes verringern. In der Fig. 3 ist ein Querschnitt durch die Blutpumpe 1 der Fig. 2 dargestellt. Im Querschnitt ist die Zylinderform des Dorns 15 deutlich erkennbar. Zwischen dem Rotor 18, insbesondere einer Innenfläche des Rotors 18 und einer Außenfläche des Dorns 15, befindet sich der radiale Spalt 23, welcher derart dimensioniert ist, dass bei einer Rotation des Rotors 18 ein Blutfilm in dem Spalt ausgebildet wird, welcher zwischen den beiden Oberflächen ein hydrodynamisches Lager ausbildet.

Vom Rotor 18 erstreckt sich eine Beschaufelung 20 und eine weitere Beschaufelung 20'. Die Beschaufelungen 20 und 20' sind jeweils eine Wendel. Diese umlaufen den Rotor 18 in radialer Richtung helixartig bzw. helixförmig. Es ist deutlich zu erkennen, dass die Breite B der Wendel 20' deutlich kleiner ist als der Abstand zwischen den beiden Wendeln 20 und 20'. Den Rotor außen umlaufend ist in einem Hohlraum 24 der Stator 22 angeordnet, welcher den Rotor 18 in Rotation versetzt. Alternativ zu einer Anordnung des Stators 22 in einem Hohlraum 24 des Gehäuses kann der Stator in einigen Ausführungsbeispielen auch innerhalb des Dorns angeordnet sein.

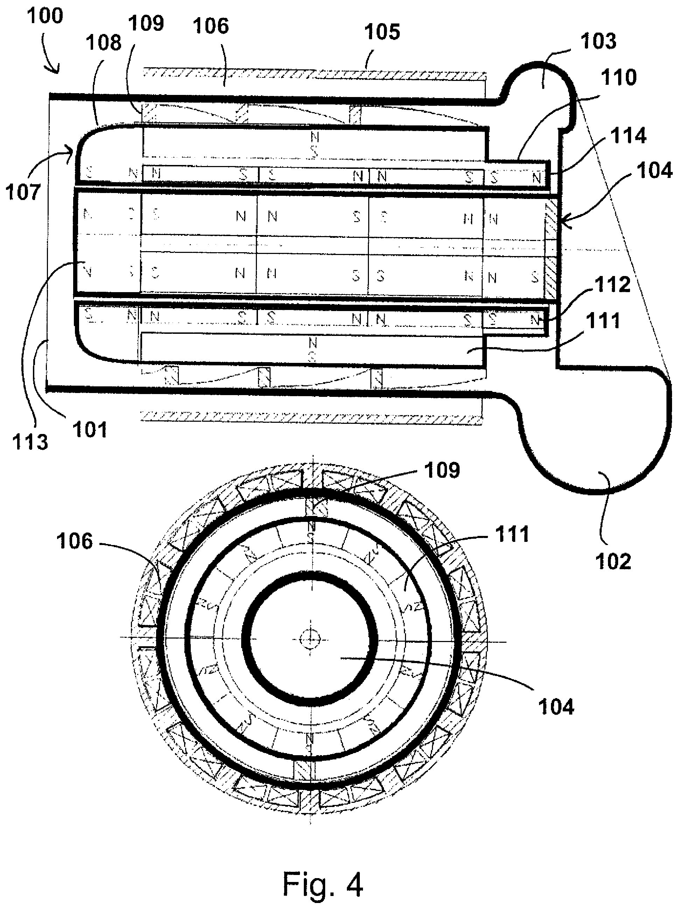

Anhand der Fign. 4 bis 8 soll auf verschiedene Ausführungsformen einer Pumpe gemäß der vorliegenden Anmeldung eingegangen werden. In der Fig. 4 ist ein Querschnitt durch eine Blutpumpe 100 dargestellt, welche einen distalen Einlass 101 sowie einen proximalen Auslass 102 umfasst. Der proximale Auslass 102 ist dabei das Ende einer sich im Querschnitt vergrößernden Spiralkammer, welche schneckenförmig verläuft. Dies ist beispielsweise anhand des kleineren Querschnitts 103 erkennbar. Vom proximalen Ende zum distalen Einlass hin erstreckt sich ein Dorn 104. Zwischen dem Einlass 101 und der Spiralkammer erstreckt sich ein zylinderförmiger Hohlraum, welcher lediglich durch den Dorn 104 unterbrochen wird. Den Hohlraum umlaufend ist ein Gehäuse 105 angeordnet, wobei sich ein Stator 106 in dem Gehäuse befindet. Auf den Dorn bzw. Zapfen 104 aufgesteckt befindet sich ein Rotor 107. Der Rotor umfasst einen distalen Abschnitt 108 mit einer Wendel 109. Des Weiteren umfasst der Rotor 107 einen proximalen Abschnitt 110, welcher keine Beschaufelung aufweist und welcher distal des Spiralauslasses beginnt und sich bis zum proximalen Ende des Rotors hin erstreckt. Damit der Rotor in Drehung versetzt werden kann, umfasst dieser Permanentmagnete 111, welche radial magnetisiert sind. Hiervon unterschieden umfasst der Rotor weitere Permanentmagnete 112, welche eine axiale Magnetisierung besitzen. Zu den axial magnetisierten Permanentmagneten 112 korrespondierend sind im Dorn 104 ebenfalls axial orientierte Permanentmagnete 113 angeordnet, welche im Zusammenspiel mit den Permanentmagneten 112 ein passives magnetisches Axiallager ausbilden. Die Magnetisierung der Permanentmagnete 112 ist entgegen der Magnetisierung der Permanentmagnete 113 ausgerichtet. Hierdurch wird bewirkt, dass beim Auslenken des Rotors, beispielsweise in distaler Richtung (d. h. nach links), die magnetische Anziehung zwischen den Polen den Rotor in die eingezeichnete Position zurückzusetzen versucht. Da der Rotor unter Rotation Blut vom Einlass in Richtung des Auslasses fördert, versucht der Druck der Flüssigkeit, den Rotor in distaler Richtung hinauszudrücken. Die Anordnung der Permanentmagnete 112 und 113 verhindert dies.

Die Permanentmagnete 112 bzw. 113 können als Ringmagnete ausgebildet sein. So können beispielsweise die Permanentmagnete 112 Zylinderhülsen sein, deren oberes Ende einen Pol und deren unteres Ende den entgegengesetzten Pol des Magnets bilden.

Im Spalt 114 zwischen dem proximalen Ende des Spiralauslasses, dem Dorn und dem Rotor wird bei Rotation des Rotors ein Blutfluss angeregt, welcher als hydrodynamisches Lager wirkt. Somit umfasst die Blutpumpe 100 keine aktiven Lager, sondern lediglich passive Lager. Im vorliegenden Beispiel besitzt die Beschaufelung 109 des Rotors 107 keinerlei Magnetisierung. In einigen Ausführungsbeispielen kann jedoch vorgesehen sein, radial außerhalb der Beschaufelung einen Ring (magnetisiert oder unmagnetisiert) anzuordnen, welcher als Dämpfungsglied zwischen einer Außenseite des Rings und einer Innenseite des Gehäuses bzw. des zylinderförmigen Hohlraums und/oder als Teil des axialen, passiven Lagers dient. In einer weiteren Ausführungsform kann der an der Beschaufelung befestigte und diese umlaufende Ring derart bemessen sein, dass ein hydrodynamisches Lager zwischen dem Ring und einer Innenwand des Einlasses gebildet wird. Erst ab einer entsprechend kleinen Spaltbreite überwiegen die Lagerkräfte, d. h., kann diese Konstruktion alleine den rotierenden Rotor radial lagern. Bei größeren Spaltbreiten reichen die Tragkräfte nicht aus, und es müssen weitere Lager, beispielsweise magnetische Lager, vorhanden sein, um den Rotor radial zu lagern.

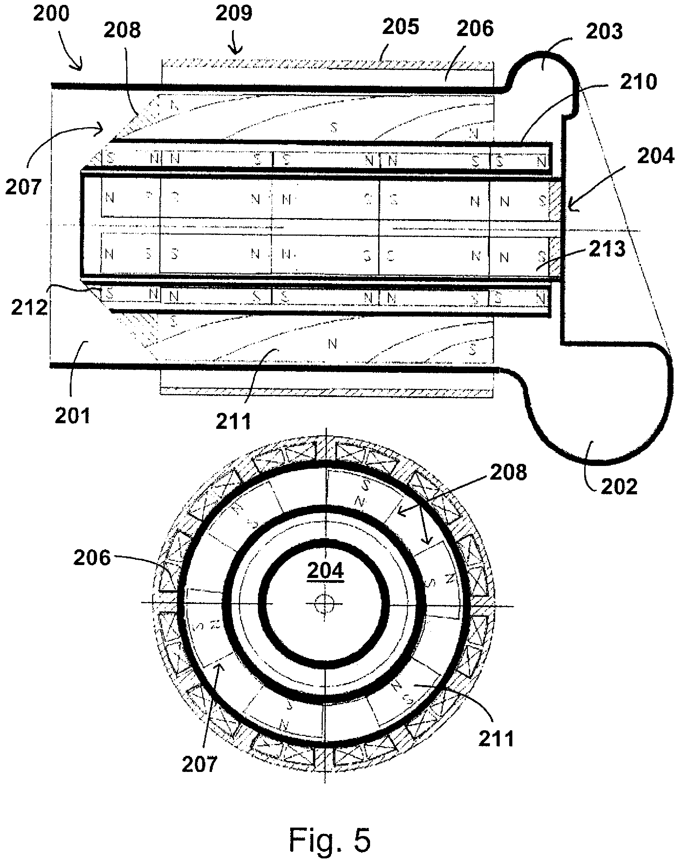

Eine weitere Blutpumpe 200 ist in der Fig. 5 dargestellt. Auch diese umfasst einen distalen Einlass 201 sowie einen proximalen Ausfass 202. Der proximale Auslass 202 hat im Vergleich zum Beginn der Spiralkammer 203 eine erweiterte Querschnittsfläche. Der Dorn 204 entspricht im Wesentlichen dem Dorn 104 der Blutpumpe 100. Dies gilt ebenso für das Gehäuse 205 und den in dem Hohlraum 206 angeordneten Stator. Der Rotor 207 unterscheidet sich von dem Rotor 107. Auch jener Rotor 207 ist derart konfiguriert, dass die Beschaufelung 208 sich lediglich im distalen Abschnitt 209 befindet, welcher distal des proximalen Auslasses 202 liegt. Das proximale Ende 210 des Rotors ist nicht mit einer Beschaufelung versehen. Um den Rotor in Rotation zu versetzen, umfasst der Rotor Permanentmagnete 211, welche sich in der Beschaufelung 208 befinden. Weiterhin umfasst der Rotor 207 axial magnetisierte Permanentmagnete 212, welche im Zusammenwirken mit den zwar ebenfalls axial, jedoch entgegengesetzt magnetisierten Permanentmagneten 213 des Dorns 204 ein passives magnetisches Axiallager ausbilden. Auch in diesem Ausführungsbeispiel versucht die mittels des Rotors geförderte Flüssigkeit den Rotor in die distale Richtung zu drücken. Die Orientierung der Permanentmagnete 212 bzw. 213 sorgt dafür, dass der Rotor in seiner Ausgangsposition gehalten wird. Als Beispiel der Beschaufelung der Blutpumpe 200 kann die Beschaufelung der Blutpumpe, welche in der US 2010/0069847 A1 offenbart ist, gesehen werden. Im Gegensatz zu dem dort gezeigten Rotor soll der Rotor gemäß dieser Anmeldung im Inneren einen Hohlraum aufweisen, um auf den Dorn gesteckt werden zu können. Die Ausführungsformen der Blutpumpe der Fign. 4 und 5 zeigen im Wesentlichen einen zylinderförmigen Dorn, auf welchem ein zylinderhülsenförmiger Rotor angeordnet ist, auf dessen Außenseite wiederum eine Beschaufelung angeordnet ist.

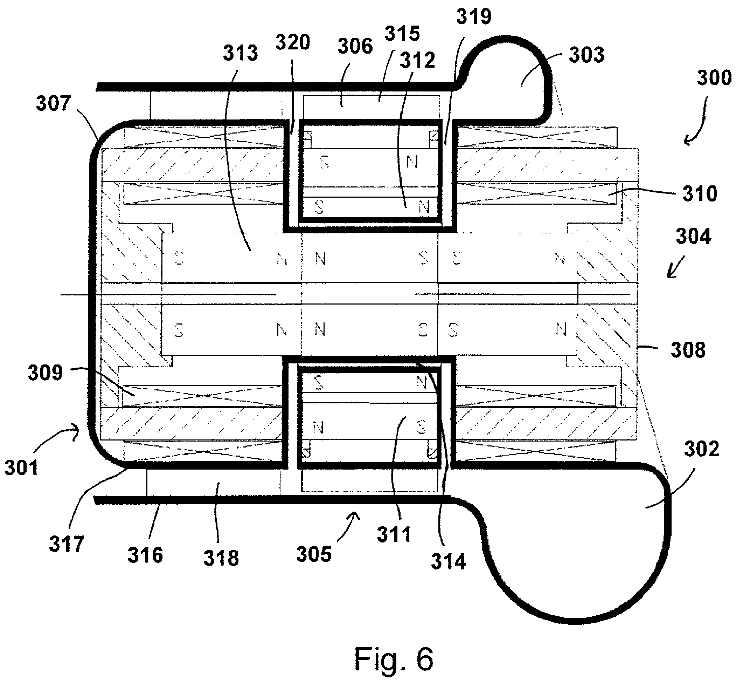

Bei der Blutpumpe 300 der Fig. 6 wird eine andere Konstruktion gewählt. Die Blutpumpe 300 umfasst einen distalen Einlass 301 sowie einen proximalen Auslass 302. Der proximale Auslass 302 weist im Gegensatz zur Spiralkammer 303 einen vergrößerten Querschnitt auf. Vom proximalen Ende zum distalen Einlass hin erstreckt sich ein Dorn 304, welcher eine ringförmige Ausnehmung 305 umfasst, in welcher ein Rotor 306 angeordnet ist. Der Dorn 304 kann dabei beispielsweise aus einem distalen Abschnitt 307 und einem proximalen Abschnitt 308 aufgebaut sein, wobei der distale Abschnitt 307 im Wesentlichen bis zu der ringförmigen Ausnehmung 305 reicht. Der distale Abschnitt 307 kann beispielsweise erst dann aufgeschraubt werden, wenn der Rotor 306 in die ringförmige Ausnehmung 305 eingesetzt wurde.

Im Dorn 304 sind Stator-Komponenten 309 sowie 310 angeordnet, welche den Rotor in Rotation versetzen können. Hierfür umfasst der Rotor Permanentmagneten 311, die derart ausgerichtet sind, dass sie durch den Stator mit einer Kraft beaufschlagt werden können. Ferner umfasst die Blutpumpe 300 weitere Permanentmagneten 312 sowie 313, welche axial ausgerichtet sind und im Zusammenspiel ein passives axiales Magnetlager bilden. In radialer Richtung ist zwischen einer Innenseite des Rotors und einer Außenseite der ringförmigen Ausnehmung ein Spalt 314 vorhanden, in welchem ein Blutfilm ausgebildet werden kann, sobald der Rotor in Bewegung versetzt wurde. Somit bildet der Spalt ein hydrodynamisches Gleitlager. Der Rotor kann dabei derart beschaffen sein, dass die Zylinderhülsenform im Wesentlichen radial bis zum radialen Umfang des Dorns außerhalb der ringförmigen Ausnehmung reicht. Somit erstreckt sich nur die Beschaufelung 315 in den zwischen der Gehäuseinnenwand 316 und dem radialen Außendurchmesser 317 des Dorns erstreckenden Innenraum 318. Alternativ zu der passiven axialen Magnetlagerung können die Spalte 319 bzw. 320 derart dimensioniert sein, dass diese ebenfalls ein axiales hydrodynamisches Gleitlager ausbilden. In diesem Fall können die Permanentmagneten 312 bzw. 313 entfallen. Alternativ kann die axiale Lage des Rotors 306 auch mittels einer aktiven axialen Lagerung geregelt werden. Da die Beschaufelung 315 radial außerhalb der Ausnehmung angeordnet ist, kann das Fluid effektiv in dem Spalt 318 gefördert werden.

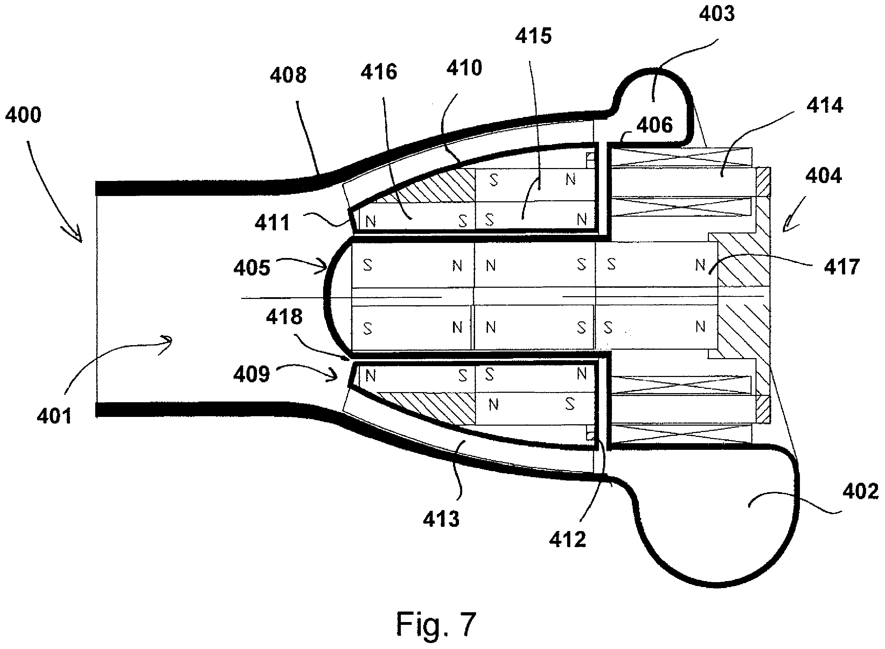

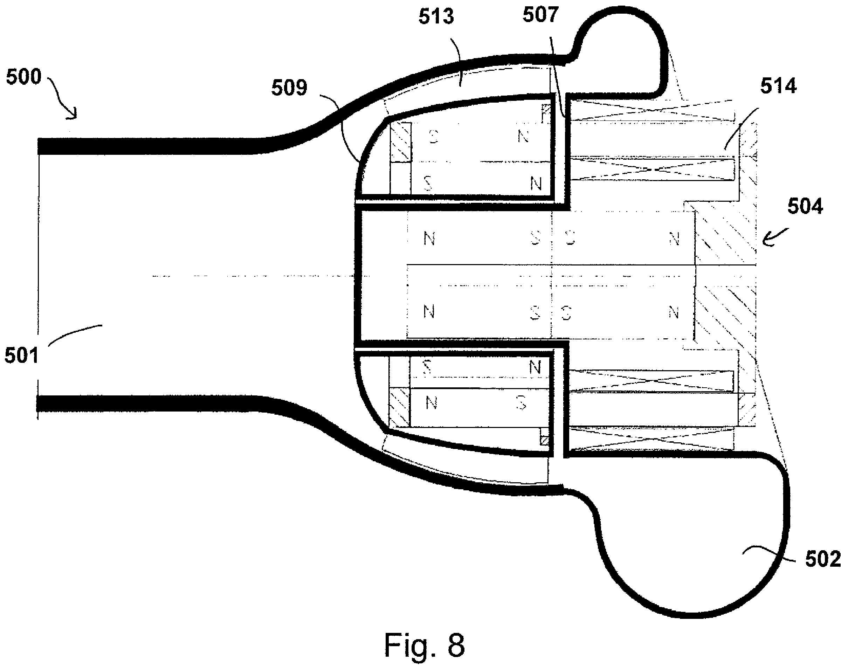

Weitere Ausführungsformen sind in den Fign. 7 und 8 dargestellt. Die Blutpumpe 400 umfasst einen distalen Einlass 401 sowie einen proximalen Auslass 402. Der proximale Auslass 402 ist mit der Spiralkammer 403 verbunden und weist in diesem Ausführungsbeispiel einen vergrößerten Querschnitt auf. Vom proximalen Ende zum distalen Einlass hin erstreckt sich ein Dorn 404, welcher einen distalen Abschnitt 405 und einen proximalen Abschnitt 406 umfasst, die über eine Schulter 407 miteinander gekoppelt sind. Die Schulter 407 kann auch als Stufe bezeichnet werden. Die Gehäusewandung 408 der Blutpumpe 400 weitet sich im Bereich des Rotors 409 vom distalen Einlass zum proximalen Auslass hin auf. Obgleich der Dorn 404 im Wesentlichen eine Zylinderform aufweist, besitzt der radial äußere Umfang des Rotors eine radiale Aufweitung 410. Im Vergleich zu Radialpumpen ist die Aufweitung jedoch so gestaltet, dass die größte Steigung der Aufweitung am distalen Ende 411 des Rotors 409 liegt und die geringste Krümmung des Rotors 409 am proximalen Ende 412 liegt. Es ist deutlich zu erkennen, dass das proximale Ende des Rotors an der Schulter 407 anliegt. Der äußere Umfang des Rotors 409 am proximalen Ende 412, ohne die Beschaufelung 413 betrachtet, stimmt im Wesentlichen mit der radialen Erstreckung des proximalen Abschnitts 406 des Dorns 404 überein. Bei der Beschaufelung kann es sich beispielsweise um eine Wendel oder eine andere, aus dem Stand der Technik für VADs bekannte Beschaufelung handeln, wie diese beispielsweise in den Ausführungsbeispielen der Fign. 4 oder 5 beschrieben wurde. Der distalste Punkt des Dorns 404 ist als Kugelsegmentoberfläche ausgebildet. Dies soll die Strömung des Bluts um den Dorn herum verbessern. Im proximalen Abschnitt 406 des Dorns 404 ist ein Stator 414 angeordnet, welcher derart konfiguriert ist, dass er mit den Permanentmagneten 415 des Rotors 409 interagiert und den Rotor somit in Drehung versetzen kann. Die axiale Lagerung kann beispielsweise mittels einer Kombination von axial magnetisierten Permanentmagneten 416 bzw. 417 bewirkt werden. In axialer Richtung wirkt zudem die Gehäusewand 408 in einigen Ausführungsbeispielen als Teil eines hydrodynamischen Lagers. Dies hängt im Wesentlichen von der der Gehäusewand gegenüberliegenden Fläche der Beschaufelung und dem Abstand der Beschaufelung von der Gehäusewand ab. Wird der Rotor 409 in distaler Richtung bewegt, besteht die Gefahr, dass die Beschaufelung 413 an der Gehäusewand 408 in distaler Richtung anschlägt, Insbesondere bei einer Wendel als Beschaufelung ist zu vermuten, dass die hydrodynamischen Kräfte zwischen der Wendelbeschaufelung und der Gehäusewand nicht ausreichen, um ein Anstoßen zu verhindern. Von daher wirken die Permanentmagneten 416 und 417 als alleinige axiale Lagerung. Eine radiale Lagerung wird durch den Spalt 418 zwischen dem Dorn 404 und dem Rotor 409 bewirkt. Selbiges gilt für den Spalt zwischen dem Rotor 409 und der Schulter 407, welcher mit dem Spalt 418 fluidisch verbunden ist.

Die Beschaufelung 413 erstreckt sich über die gesamte Länge des Rotors, von distaler in proximaler Richtung gesehen.

Die Fig. 8 zeigt eine Blutpumpe 500, welche im Wesentlichen der Blutpumpe 400 der Fig. 7 entspricht. Auch die Blutpumpe 500 weist einen distalen Einlass 501 sowie einen proximalen Auslass 502 auf. Ferner ist ein Dorn 504 mit einer Schulter 507 vorhanden. Der Rotor 509 ist, anders als im Ausführungsbeispiel der Fig. 7, nicht über seine gesamte Länge mit einer Beschaufelung 513 versehen. So beginnt die Beschaufelung 513 in einem Abstand vom distalen Anfang des Rotors 509. Auch bei dem Ausführungsbeispiel der Fig. 8 ist der Rotor 509 derart beschaffen, dass die Krümmung der radialen Außenflächen vom distalen Einlass zum proximalen Ausfass hin abnimmt. Ein Stator 514 versetzt den Rotor anhand von Permanentmagneten in Rotation. Ferner ist eine axiale Lagerung vorhanden, welche im Wesentlichen der axialen Lagerung der Fig. 7 entspricht. Der Dorn 504 ist an seinem distalen Ende abgeflacht. Der Rotor schließt bündig mit dem stromaufwärts gelegenen Ende des Dorns 504 ab.

Weitere Ausführungsbeispiele ergeben sich für den Fachmann in naheliegender Weise.

## Patentansprüche

1. Blutpumpe (1; 100; 200; 300; 400; 500), vorzugsweise zur Unterstützung eines Herzens, wobei die Blutpumpe ein Gehäuse mit einem stromaufwärts gelegenen Einlass (11; 101; 201; 301; 401), einem stromabwärts gelegenen Auslass (12; 102; 202; 302; 402) sowie einem zwischen dem Einlass und dem Auslass angeordneten Dorn (15; 104; 204; 304; 404; 504) umfasst und koaxial auf dem Dorn ein Rotor (18; 108; 208; 306; 409; 509) mit einer Achse und Beschaufelung (20; 109; 209; 315; 413; 513) drehend angeordnet ist, wobei der Rotor auf dem Dorn in einer axialen Richtung magnetisch gelagert ist,
wobei
der Auslass stromabwärts eines stromabwärts gelegenen Endes der Beschaufelung angeordnet ist.

2. Blutpumpe nach Anspruch 1, wobei der Auslass vollständig stromabwärts des stromabwärts gelegenen Endes der Beschaufelung angeordnet ist.

3. Blutpumpe nach einem der vorhergehenden Ansprüche, wobei der Auslass eine gegenüber der Achse des Rotors um einen Winkel von mehr als 45°, vorzugsweise zwischen 80° und 100°, geneigte Auslassrichtung vorgibt.

4. Blutpumpe nach Anspruch 3, wobei der Auslass eine Volute (203; 303; 403) umfasst.

5. Blutpumpe nach einem der vorhergehenden Ansprüche, wobei der Rotor eine Hülse umfasst und die Beschaufelung entlang eines äußeren Umfangs der Hülse angeordnet ist.

6. Blutpumpe nach Anspruch 5, wobei die Hülse und der Dorn derart zueinander ausgerichtet sind, dass ein hydrodynamisches Lager zwischen einem inneren Umfang der Hülse und einer Oberfläche des Dorns gebildet ist.

7. Blutpumpe nach einem der Ansprüche 5 oder 6, wobei die Hülse eine Zylinderhülse ist.

8. Blutpumpe nach einem der Ansprüche 5 bis 7, wobei in der Hülse und im Dorn Permanentmagnete eines axialen magnetischen Lagers angeordnet sind.

9. Blutpumpe nach einem der Ansprüche 5 bis 7, wobei in der Hülse und im Gehäuse Permanentmagnete (112,113; 212, 213; 312, 313; 416, 417) eines axialen magnetischen Lagers angeordnet sind.

10. Blutpumpe nach einem der vorhergehenden Ansprüche, wobei die Beschaufelung mindestens eine Wendel umfasst.

11. Blutpumpe nach einem der vorhergehenden Ansprüche, wobei ein Stator zum Antrieb des Rotors vorhanden ist und der Stator im Dorn und/oder im Gehäuse, den Rotor umlaufend und/oder proximal des Rotors angeordnet ist.

12. Blutpumpe nach einem der vorhergehenden Ansprüche, wobei der Dorn im Wesentlichen zylinderförmig ist.

13. Blutpumpe nach einem der Ansprüche 1 bis 11, wobei der Dorn im Wesentlichen eine ringförmige Ausnehmung (305) umfasst, in welcher der Rotor drehbar gelagert ist.

14. Blutpumpe nach Anspruch 13, wobei die Beschaufelung radial außerhalb der Ausnehmung angeordnet ist.

15. Blutpumpe nach einem der vorhergehenden Ansprüche, wobei der Rotor derart konfiguriert ist, dass das stromabwärts gelegene Ende der Beschaufelung weiter stromaufwärts angeordnet ist als ein stromabwärts gelegenes Ende des Rotors.

16. Blutpumpe nach einem der vorhergehenden Ansprüche, wobei der Dorn einen ersten, distalen Abschnitt (405) zur Aufnahme des Rotors und einen zweiten, proximal des ersten Abschnitts gelegenen Abschnitt (406) umfasst und zwischen dem ersten und zweiten Abschnitt eine Schulter (407) einen Anschlag für ein proximales Ende des Rotors bildet.

17. Blutpumpe nach Anspruch 16, wobei ein Durchmesser des zweiten Abschnitts im Wesentlichen einem äußeren Durchmesser des Rotors ohne Beschaufelung gleich ist.

18. Blutpumpe nach einem der vorhergehenden Ansprüche, wobei der Rotor in radialer Richtung hydrodynamisch gelagert ist.
